# EUROPEAN PATENT SPECIFICATION

(11) **EP 3 393 279 B1**
(45) Date of publication and mention of the grant of the patent: **16.08.2023**
(21) Application number: 16809058.7
(22) Date of filing: 09.12.2016
(51) Int. Cl.: A24F 40/40, A24F 40/10

(54) **FLEXIBLE AEROSOL-GENERATING DEVICES**
FLEXIBLE AEROSOLERZEUGENDE VORRICHTUNGEN
DISPOSITIFS DE GÉNÉRATION D'AÉROSOL FLEXIBLE

(30) Priority: 24.12.2015 EP 15202727
(43) Date of publication of application: 31.10.2018
(73) Proprietor: Philip Morris Products S.A., 2000 Neuchâtel (CH)
(72) Inventor: BATISTA, Rui Nuno, 1110 Morges (CH); HEDARCHET, Stephane Antony, 1009 Pully (CH)
(74) Representative: Nevett, Duncan
(86) International application number: PCT/EP2016/080478
(87) International publication number: WO 2017/108452

(56) References cited:
- KR-A- 20120 089 546
- US-A1- 2002 008 122
- US-A1- 2015 264 978

## Description

The present invention relates to an aerosol-generating device and components for an aerosol-generating device. Aspects of the invention find particular application to electrically operated aerosol-generating devices, such as electrically operated smoking devices.

Aerosol-generating devices are known and may consist of a device portion comprising a battery and control circuitry, an electrically operated vaporizer portion, and a consumable portion comprising an aerosol-forming substrate. A cartridge comprising both an aerosol-forming substrate and vaporizer is sometimes referred to as a "cartomiser". The cartridge portion typically comprises not only the aerosol-forming substrate and an electrically operated heating element, but also a mouthpiece. The "mouthpiece" refers to a portion of the aerosol-generating device that includes a part which is placed into a user's mouth. The user sucks on the mouthpiece in use to draw aerosol into their mouth. The vaporizer is typically a heating element for example comprising a coil of heater wire wound around an elongate wick soaked in liquid aerosol-forming substrate. In some examples, the aerosol-forming substrate may comprise a solid aerosol-forming substrate, such as granules or shreds of material, for example tobacco-containing material. The vaporizer may in such an example include a heating element which is arranged to heat the solid aerosol-forming substrate. In some known examples, the aerosol-forming substrate is an aerosol-forming liquid, sometimes referred to as an e-liquid.

Aerosol-generating devices are usually portable. Users often carry their device with them and when not in use may store their device in clothes pockets for ease of access. Such storage of the aerosol-generating devices may increase the risk of damage to the aerosol-generating devices. Some components of aerosol-generating devices may be fragile or are unable to withstand forces such as bending forces applied to them when being carried by the user. For example the movements of the user may damage the device being stored in the user's pocket. Although hardened carrying cases may be used to protect the aerosol-generating devices, such cases are necessarily bigger than the article they protect and may make the aerosol-generating device undesirably large or hard for comfortable pocket storage. It would be beneficial to provide comfortable storage of aerosol-generating devices, in particular those having electrical components, while reducing the risk of damage to the aerosol-generating devices.

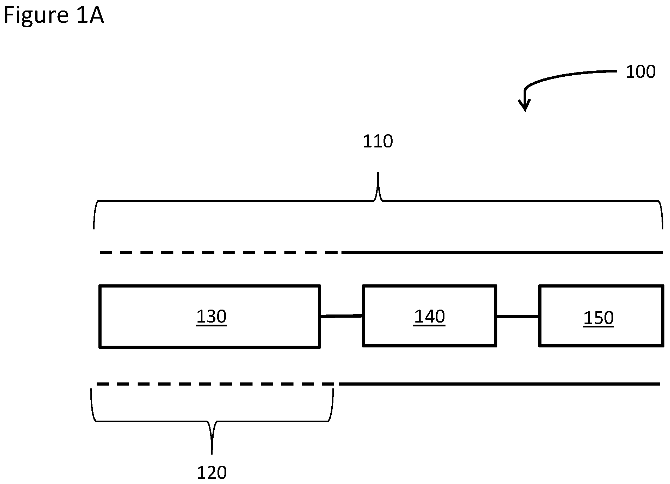

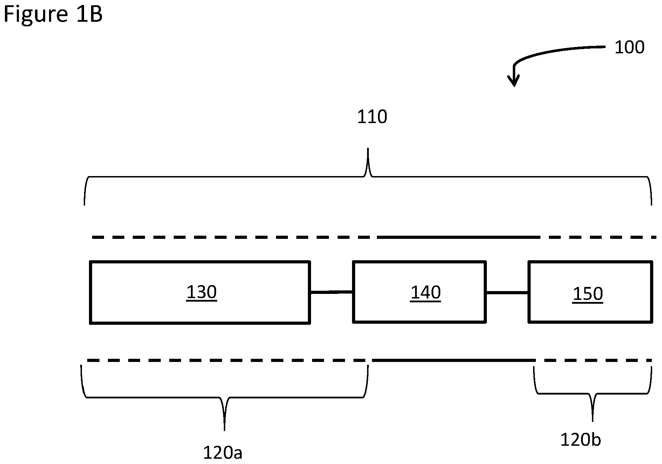

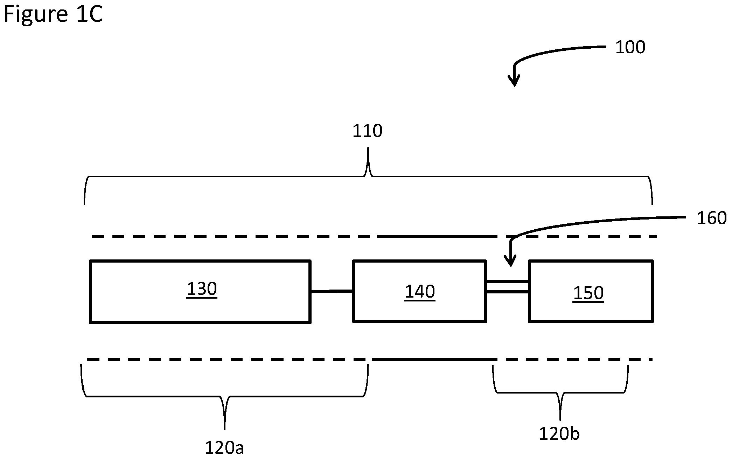

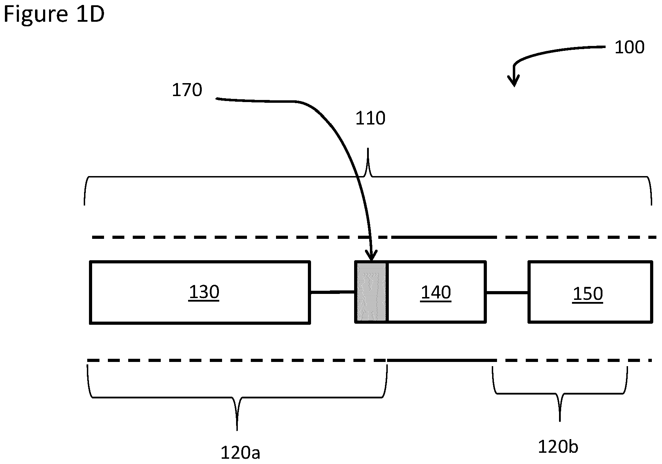

US 2015/0264978 A1 discloses a wearable electronic simulated smoking device comprising an elongate tubular member having at least a portion thereof being reversibly bendable for said tubular body to at least partially encompass a portion of a user's body. In FIG. 1 there is shown a wearable electronic simulated smoking device 100' that has a configuration where at least a portion of the tubular body 110 has a fixed contour and at least another portion is bendable to change the contour thereof. The wearable electronic simulated smoking device 100' has a tubular body formed by bendable portions 112*a*, 112*b* and the portions 114*a*, 114*b* having a fixed arcuate contour. The bendable portions 112*a* and 112*b* respectively extend from the distal ends 116*a* and 116*b* of the arcuate portions 114*a* and 114*b*. The operational components of wearable electronic simulated smoking device 100' are distributed within the internal bore 1101*a*, 1101*b* of the tubular portions 114*a* and 114*b*. The portions 114*a* and 114*b* are joined by a mouthpiece connector 118 that has a connector body 117 from which the mouthpiece 120 extends. When a user wishes to encompass a portion of their body, such as their neck, wrist or finger, with the electronic simulated smoking device 100', the user bends the portions 112*a* and 112*b* outwardly, and passes the tubular body 110 around the selected portion of the user's body. Once positioned, the user either releases the bendable portions 112*a* and 112*b* to return to their original arcuate contour and at least partially encompass the selected portion of the user's body, when bendable portions 112*a* and 112*b* have an elastic property, or manually bend the bendable portions 112*a* and 112*b* back into an arcuate contour sufficient to at least partially encompass the selected portion of the user's body and maintain the electronic simulated smoking device 100' thereat.

US 2002/0008122 A1 describes a discharge apparatus for media that, in one embodiment, is actuated by bending part of a casing of the apparatus relative to a main part.

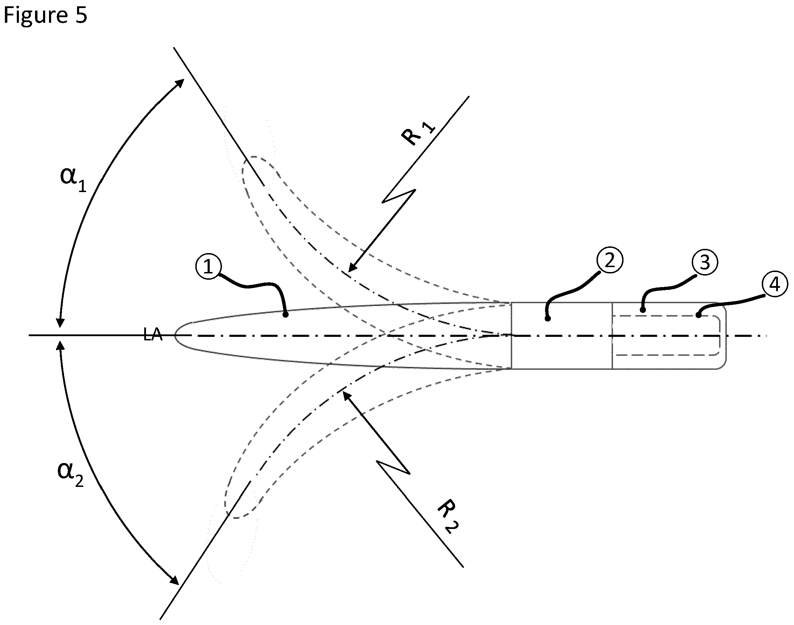

KR 2012-0089546 A discloses an inhaling apparatus comprises an inhaling apparatus case and a joint member which bends the inhaling apparatus case while connecting each unit of the inhaling apparatus case. The inhaling apparatus case is composed of a plurality of division members. The joint member is arranged between a plurality of division members. A plurality of division members can be bent. The joint member may comprise a crinkle shape or a rotary hinge. In the suction device 500 shown in Figure 5, each component constituting the suction device case 500, for example, the electric supply member 513, the control member 514, the suction detection switch 515, the vaporizing member 518, the liquid receiving member 519 and the like are configured such that a part of the suction device case 511 can be bent by an external force. In order that it bends, a portion 530 of the suction device case 511 has a crinkle shape. The liquid receiving member 519 of the suction device 500 is accommodated in the portion 530 of the suction device case 511 having the crinkle shape. The portion 530 of the suction device case 511 bends with the external force of the user.

The present invention is defined in the appended claims.

According to a first aspect an aerosol-generating device includes a housing and a flexible device component disposed in the housing. The housing has a length and defines a rigid portion along at least a portion of the length of the housing and a flexible portion along at least a portion of the length of the housing. The flexible portion is elastically deformable or elastically deflectable and has a relaxed or unloaded configuration and is configured to adopt a flexed or deflected configuration. When the housing is in a relaxed or un-bent configuration, the flexible device component is at least partially longitudinally aligned with the flexible portion of the housing.

The flexible portion of the housing is elastically deformable or elastically deflectable. In other words, the flexible portion is configured to adopt the relaxed or unloaded configuration naturally or originally when no external force or load is applied to the flexible portion. The flexible portion of the housing is substantially linear in the relaxed or unloaded configuration. The flexible portion defines a longitudinal axis in the relaxed or unloaded configuration. The flexible portion is configured to adopt the flexed or deflected configuration under the influence of an external force or load. The flexible portion is configured to return to the relaxed or unloaded configuration from the flexed or deflected configuration when the external force or load is removed from the flexible portion.

The housing also defines a rigid portion along at least a portion of the length of the housing. The rigid portion may be substantially inflexible.

In another aspect, the flexible portion of the housing defines a longitudinal axis in the relaxed or unloaded configuration. When the flexible portion of the housing is in the flexed or deflected configuration, at least a portion of the flexible portion of the housing deflects from the longitudinal axis by an angle of about 10 degrees or more, about 20 degrees or more, or about 30 degrees or more. When the flexible portion of the housing is in the flexed configuration, at least a portion of the flexible device component remains substantially longitudinally aligned with at least a portion of the flexible portion of the housing being deflected.

In another aspect, the flexible portion of the housing has a first end, an opposing second end, and a middle between the first end and the second end. The middle is about equal distance from the first end as it is from the second end. When the housing is in the flexed configuration, the flexible portion of the housing defines a radius of curvature at the middle of the flexed portion. The radius of curvature may be about 40 mm to about 120 mm, about 50 mm to about 100 mm, or about 60 mm to about 90 mm.

In another aspect, the flexible device component includes one or more of: a flexible power supply, which may include a flexible battery; flexible control circuitry; a flexible mouthpiece; a flexible storage component; and a flexible fluid flow passage component.

In another aspect, the aerosol-generating device includes a rigid portion which may include a heating element.

In another aspect, the aerosol-generating device includes a rigid portion which may include a mouthpiece. The mouthpiece may be a rigid mouthpiece. The rigid mouthpiece may be substantially inflexible. The rigid portion may be at a first end of the aerosol-generating device and comprise a rigid mouthpiece and the flexible portion may be arranged at a second, opposite end of the aerosol-generating device.

The aerosol-generating devices described herein may provide one or more advantages over previously available or described smoking articles and aerosol-generating devices. For example, providing an aerosol-generating device with flexible components, for example flexible electrical components, and a flexible housing may reduce the risk of breaking while stored in a pocket. In addition, the flexible components and flexible housing may permit the aerosol-generating device to bend with the user or user's clothing during storage, providing greater comfort for the user when the aerosol-generating device is stored in next-to-body pockets or locations. The decreased fragility of an aerosol-generating device with flexible components and a flexible housing may also decrease the risk that an aerosol-generating device will be damaged when carried in a bag such as a purse, satchel, or backpack. These and other advantageous will be readily understood upon reading the disclosure presented herein.

The aerosol-generating device includes a housing and a flexible device component disposed in the housing. The flexible device component may include, for example, a flexible power supply, a flexible storage component, a flexible fluid flow passage component, a flexible control circuitry, and other suitable flexible components.

At least a portion of the housing of the aerosol-generating device of the invention is flexible. Device components are disposed in the housing. Some of the device components may be flexible. A flexible device component is disposed in the housing. Preferably, one or more flexible components are disposed within a flexible portion of the housing. Preferably, the flexible portion of the housing defines the amount that the device may flex to protect underlying device components.

A housing of an aerosol-generating device of the invention may extend the full length of the aerosol-generating device or only a portion of the length of the aerosol-generating device. The flexible portion of the housing may be a continuous portion of the housing or a discontinuous portion of the housing.

At least a portion of the housing is flexible. For example, the length of the flexible portion of the housing may be about 25% to about 70%, about 25% to about 80%, about 25% to about 90% of the length of the housing.

Various components of the aerosol-generating device may be disposed within the housing including, for example, one or more of a power supply including, for example, a battery; control circuitry; a vaporizer including, for example, a heating element; a storage component including, for example, an aerosol-forming substrate; a fluid flow passage component; a vaporizer; a mouthpiece; or other component.

Various device components of the aerosol-generating device may be disposed within the flexible portion of the housing including, for example, one or more of a power supply including, for example, a battery; control circuitry; a vaporizer including, for example, a heating element; a storage component including, for example, an aerosol-forming substrate; a fluid flow passage component; a vaporizer; a heating element; a mouthpiece; or other suitable components. As further discussed below, one or more components of the aerosol-generating device disposed within the flexible portion of the housing may be flexible.

Components of the aerosol-generating device may be disposed within a rigid portion of the housing. For example, at least one of a heating element and a storage component are preferably disposed in a rigid portion of the housing. The terms "rigid" and "inflexible" refer to an element or a portion being less flexible than the "flexible" element or portion.

An aerosol-generating device of the invention may be flexible or include a flexible portion. A "flexible" aerosol-generating device, device portion, or device component is a device, device portion, or device component that may elastically bend or deflect to a certain extent upon the application of external force or load at room temperature and may return to a relaxed configuration, or may be returned to its original unloaded configuration, without any portion of the device breaking or being permanently deformed. A "relaxed configuration" of the aerosol-generating device, device portion, or a device component refers to the state of the aerosol-generating device, device portion, or device component in the absence of the application of external force or load.

An aerosol-generating device of the invention may include a rigid portion or device component. A "rigid" device portion or device component is a device portion or device component that may elastically bend or deflect to a lesser extent than a flexible device portion or flexible device component upon the application of external force or load at room temperature. The rigid device portion or device component may be inelastic. The degree of inflexibility may depend on the device part or device component.

When in a relaxed or unloaded configuration, the aerosol-generating device, a flexible portion of the housing of the aerosol-generating device, or a flexible device component of the aerosol-generating device has a longitudinal axis. When in a maximum flexed or loaded configuration, at least a portion of the aerosol-generating device, the flexible portion of the housing of the aerosol-generating device, or a flexible device component of the aerosol-generating device deflects from the longitudinal axis by an angle of about 10 degrees to about 100 degrees including, about 20 to about 90 degrees, or about 30 to about 80 degrees. The deflection from the longitudinal axis may be, for example, at least about 10 degrees, at least about 15 degrees, at least about 20 degrees, at least about 25 degrees, at least about 30 degrees, at least about 35 degrees, at least about 40 degrees, at least about 50 degrees, at least about 60 degrees, at least about 70 degrees, at least about 80 degrees, at least about 90 degrees, at least about 95 degrees. Preferably, the deflection may not exceed about 100 degrees.

As used herein, a "maximum flexed configuration" is the maximum amount that a device, housing or component may be flexed without breaking or plastically deforming the device, housing or component. Preferably, the housing is designed to resist further flexing to an extent less than the maximum flexed configuration of a flexible component disposed in the flexible portion of the housing, if there is a flexible component disposed in the flexible portion of the housing. Accordingly, the housing may resist flexing of the device to an extent that may cause an internal component to break.

A flexible portion of a device according to the invention may deflect from the longitudinal axis in a symmetrical manner or in an asymmetrical manner. For example, a portion of the device, flexible portion of the housing, or a flexible component of the device may deflect along the length of the flexible portion to the same extent in different directions or to different extents or in different directions.

The flexible portion of the housing of the aerosol-generating device has a first end, an opposing second end, and a middle between the first end and the second end, the middle is about equal distance from the first end as it is from the second end. When the flexible portion of the housing of the aerosol-generating device is in a flexed configuration, the flexible portion defines a radius of curvature at the middle of the flexed portion. The "radius of curvature" is a measure at a particular point on a curve of the radius of the circle which best approximates the curve at that point. The radius of curvature of the flexible portion of the housing may be about 40 mm to about 120 mm, about 50 mm to about 100 mm, or about 60 mm to about 90 mm. The radius of curvature may be about 80 mm. The radius of curvature may be measured when the flexible portion of the housing is in a maximum flexed configuration.

Preferably, the flexible portion of the housing defines the amount that the flexible components of the device may flex or deflect.

The housing of the aerosol-generating device may be made of any suitable material or materials. At least a portion of the housing that is flexible is made from a material or materials that permit the flexible portion of the housing to have a relaxed or un-bent configuration and to be configured to adopt a flexed configuration. The housing may comprise one, two, or more elements which may for example be releasably or non-releasably attached together.

For example, the housing may be made of one material, two materials, three materials, four materials, five materials, or more than five materials. The housing may be formed, for example, by molding or overmolding or may be assembled.

The materials forming the housing may include at least one of elastomeric compounds, polymeric compounds, elastomeric or rubber compounds except natural rubber compounds, and polyurethane based compounds. The elastomeric materials may include, for example, compounds containing ethylene propylene diene monomer (EPDM), vinyl methyl quality (VMQ) silicone, fluorovinylmethylsiloxane (FVMQ), or other appropriate material or combination of materials. The polymeric compounds may include, for example, compounds containing, for example, polypropylene (PP), polyamide (PA), fluorinated ethylene propylene (FEP), polyethylene (PE), cross-linked polyethylene (XPLE or PEX), polyether ether ketone (PEEK), or other appropriate polymeric compounds. The polymeric compounds may include thermoplastics including, for example, Crastin^{®} Polybutylene Terephthalate, Delrin^{®} Acetal Homopolymer Resin, Hytrel^{®} Thermoplastic Elastomer, and Zytel^{®} Nylon Resin, or other appropriate material or combination of materials.

The housing may further include a coating. All or some of the housing of the aerosol-generating device may further include a coating. The materials forming the coating of the housing may include, for example, elastomeric materials.

The flexible portion of the housing may include a polymeric compound and further includes a coating that includes elastomeric or rubber compounds or polyurethane based compounds. For example, the materials forming the flexible portion of the housing may include compounds including at least one of polypropylene (PP), polyamide (PA), fluorinated ethylene propylene (FEP), polyethylene (PE), and cross-linked polyethylene (XPLE or PEX) or thermoplastics including at least one of Crastin^{®} Polybutylene Terephthalate, Delrin^{®} Acetal Homopolymer Resin, Hytrel^{®} Thermoplastic Elastomer, and Zytel^{®} Nylon Resin. The materials forming the flexible portion of the housing may further include materials forming a coating including compounds including at least one of ethylene propylene diene monomer (EPDM), vinyl methyl quality (VMQ) silicone, and fluorovinylmethylsiloxane (FVMQ).

One or more flexible components of an aerosol-generating device according to the invention may be disposed in the housing. For example, an aerosol-generating device according to the invention may include a flexible power supply. A flexible power supply may be disposed in the flexible portion of the housing. The flexible power supply is preferably at least partially longitudinally aligned with at least a portion of the flexible portion of the housing. The flexible power supply may be made of any material or materials that allows the portion of the power supply that is longitudinally aligned with the flexible portion of the housing to flex at least as much as the flexible portion of the housing when the flexible housing when the flexible portion of the housing is in the flexed configuration. Such flexion or deflection occurs without damage to the flexible power supply. In addition, the flexible power supply preferably remains longitudinally aligned with the flexible portion of the housing when the flexible portion of the housing is in the flexed configuration and when the flexible portion of the housing returns to the relaxed configuration.

The flexible power supply may include at least one battery. The battery may be a rechargeable or non-rechargeable battery. A rechargeable battery may include, for example, a lithium ion battery, including for example, a lithium ion manganese oxide battery; a nickel metal hydride battery; a thin film battery, or other battery. A non-rechargeable battery may include, for example, a button cell battery, a lithium battery, or other battery. The battery may be flexible, for example, a thin film battery. The battery may be incorporated in a flexible electronics matrix.

The flexible power supply may include two or more electronic modules connected by flexible connectors. The electronic modules may be flexible or rigid. For example, the flexible power supply may include either substantially flexible or rigid electronic modules or both substantially flexible and rigid electronic modules and flexible connectors. The flexible connectors may include ribbon wires. Preferably, the electronic modules are batteries. The two or more batteries may be configured in a series, in parallel, or in a mixture of both to provide the desired voltage, capacity, or power density.

In addition or alternatively, one or more flexible components other than a flexible power supply may be disposed in a flexible portion of the housing. Examples of other flexible components include a flexible storage component, a flexible fluid flow passage component, a flexible control circuitry, and other suitable flexible components.

Preferably, an aerosol-generating device according to the invention comprises flexible control circuitry. The control circuitry may, for example, control the supply of power to a heating element. At least a portion of the flexible control circuitry may be disposed within the housing and may be at least partially longitudinally aligned with at least a portion of the flexible portion of the housing such when the housing is in the flexed configuration at least a portion of the flexible control circuitry remains longitudinally aligned with at least a portion of the flexible portion of the housing.

The control circuitry may be made of any material or materials that preferably allows the portion of the control circuitry that is longitudinally aligned with the flexible portion of the housing to flex at least as much as the flexible portion of the housing when the flexible housing when the flexible portion of the housing is in the flexed configuration. Such flexion or deflection preferably occurs without damage to the flexible control circuitry. In addition, the flexible control circuitry preferably remains longitudinally aligned with the flexible portion of the housing or the neutral axis of the housing when the flexible portion of the housing is in the flexed configuration and when the flexible portion of the housing returns to the relaxed or unloaded configuration.

The flexible control circuitry may include either a flexible printed circuit board or a rigid-flex circuit or both a flexible printed circuit board and a rigid-flex circuit. The flexible control circuitry may include two or more electronic modules connected by flexible connectors. The electronic modules may be flexible or rigid.

The aerosol-generating device according to the invention may include a flexible storage component. The flexible storage component may include an aerosol-forming substrate. The aerosol-forming substrate may include nicotine. The aerosol-forming substrate may be solid or an aerosol-forming liquid, sometimes referred to as an e-liquid. The flexible storage component may include a flexible reservoir. Examples of flexible reservoirs that may be used in an aerosol-generating device of the invention include, for example, a bag or a pouch.

The aerosol-generating device according to the invention may include a flexible fluid flow passage component. The fluid flow passage component may include a flexible tube. The fluid flow passage component may be made of any suitable material or materials. For example, the fluid flow passage component may be made of a polymeric material.

The aerosol-generating device of the invention may include a heating element. The aerosol-generating device may include more than one heating element, or, for example, two, or three, or four, or five, six, or more than six heating elements. One or more heating elements may be an inductive heating element. One or more heating elements may be a conductive heating element. One or more of the heating elements may be disposed within a rigid portion of the housing.

The aerosol-generating device of the invention may include a mouthpiece. The mouthpiece may be disposed outside the housing of the aerosol-generating device. The mouthpiece may be disposed within the housing of the aerosol-generating device including, for example, within the flexible portion of the housing of the aerosol-generating device. Alternatively, the mouthpiece may extend from or form a portion of the housing.

The mouthpiece may be flexible, partially flexible, or rigid. The mouthpiece may include a rigid core including, for example, a metallic tube, or a flexible core. The mouthpiece may include a cap. The cap may be separate from or integrated with the housing. The cap may be flexible, partially flexible, or rigid. The mouthpiece portion may be part of a cartridge.

The aerosol-generating device may be substantially "flat" or "planar" and may have, for example, a rectangular cross section. The aerosol-generating device may be elliptical, or another shape, in cross section. The aerosol-generating device according to the invention may be substantially cylindrical in shape in cross section. The aerosol-generating device article may be substantially elongate. The aerosol-generating device may have a length and a circumference substantially perpendicular to the length.

The aerosol-generating device may have a size comparable to a conventional cigar or cigarette. The aerosol-generating device may have a total length of about 30 mm to about 150 mm. The aerosol-generating device may have an external diameter of about 5 mm to about 30 mm. The aerosol-generating device may have an external diameter of about 5 mm to about 12 mm. The aerosol-generating device may have an external circumference of about 15 mm to about 150 mm.

In one preferred configuration, the aerosol-generating device has a total length of about 45 mm. The aerosol-generating device may have an external diameter of about 7.2 mm.

Preferably, the flexible portion of the housing makes up about 25% to about 70% of the length of the aerosol-generating device, about 30% to about 70% of the length of the aerosol-generating device, about 35% to about 70% of the length of the aerosol-generating device, about 40% to about 70% of the length of the aerosol-generating device, about 45% to about 70% of the length of the aerosol-generating device, or about 50% to about 70% of the length of the aerosol-generating device.

Thus aspects of the invention provide an aerosol-generating device, preferably an electrically operated device, for example a smoking article, having a flexible portion are described. For example, the device may have a flexible component disposed within a flexible portion of a housing. Some aspects of the invention relate to flexible electrical components disposed within a flexible housing, for example for use in an aerosol-generating device. The flexible electrical components may include for example a power supply or control circuitry.

Any feature in one aspect of the invention may be applied to other aspects of the invention, in any appropriate combination. That is, any, some, or all of the features in one aspect may be applied to any, some, or all features in another aspect, in appropriate combination. It should also be appreciated that particular combinations of the various features described and defined in any aspects of the invention may be implemented or supplied or used independently.

Embodiments of the invention will be further described, by way of example only, with reference to the accompanying drawings in which:
Figure 1A shows a schematic representation of an aerosol-generating device in accordance with one configuration of the present invention; Figure 1B shows a schematic representation of an aerosol-generating device in accordance with one configuration of the present invention; Figure 1C shows a schematic representation of an aerosol-generating device in accordance with one configuration of the present invention; Figure 1D shows a schematic representation of an aerosol-generating device in accordance with one configuration of the present invention.
Figure 2A shows a schematic representation of the flexible electrical components of an aerosol-generating device in accordance with one configuration of the present invention; and Figure 2B shows a schematic representation of the flexible electrical components of an aerosol-generating device in accordance with one configuration of the present invention.
Figure 3 shows a schematic representation of the flexible electrical components of an aerosol-generating device in accordance with one configuration of the present invention.
Figure 4 shows the electrical components and the housing of an aerosol-generating device in accordance with one configuration of the present invention.
Figure 5 shows the housing of an aerosol-generating device in accordance with one configuration of the present invention.

Figure 1A shows a schematic representation of an aerosol-generating device **100** in accordance with one configuration of the present invention. As shown in Figure 1A, the aerosol-generating device **100** has a housing **110.** The electrical components **130** of the aerosol-generating device **100** are disposed within the flexible portion **120** of the housing **110.** The aerosol-generating device **100** may further include a heating element **140** and a mouthpiece **150** disposed within the housing. Alternatively, a portion of the housing may form the mouthpiece or the mouthpiece may be attached to the housing.

Figure 1B shows a schematic representation of an aerosol-generating device **100** in accordance with one configuration of the present invention. The electrical components **130** and the mouthpiece **150** of the aerosol-generating device **100** are disposed within flexible portions **120a, 120b** of the housing **110,** and the heating element **140** is disposed in a rigid portion of the housing **110.** The electrical components **130** or the mouthpiece **150** or both may be a flexible device component.

Figure 1C shows a schematic representation of an aerosol-generating device **100** in accordance with one configuration of the present invention. A flexible fluid flow passage **160** component is disposed within the flexible portion of the housing. Reference is made to the discussion above regarding Figures 1A and 1B for the numbered elements depicted in, but not specifically described regarding, Figure 1C.

Figure 1D shows a schematic representation of an aerosol-generating device **100** in accordance with one configuration of the present invention. A flexible storage compartment **170** is disposed within a flexible portion **120a** of the housing **110.** The aerosol-forming substrate may be located within the flexible storage compartment **170.** The aerosol-forming substrate may include nicotine. The aerosol-forming substrate may be liquid. Reference is made to the discussion above regarding Figures 1A, 1B, and 1C for the numbered elements depicted in, but not specifically described regarding, Figure 1D.

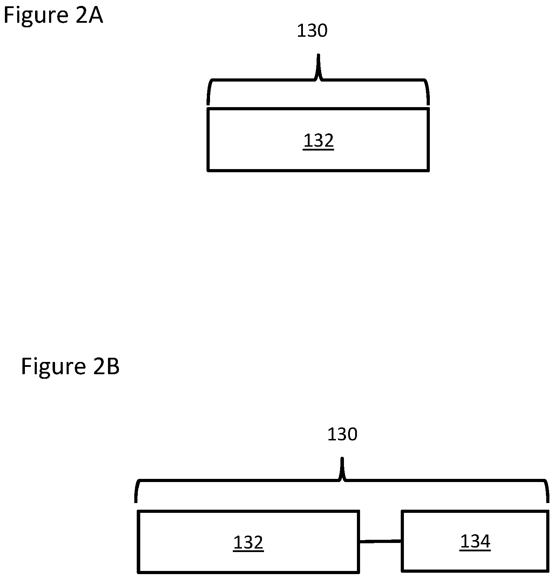

Figure 2A shows a schematic representation of the electrical components **130** of an aerosol-generating device in accordance with one configuration of the present invention including a power supply **132.** The power supply **132** may be flexible. Figure 2B shows a schematic representation of the electrical components **130** of an aerosol-generating device in accordance with one configuration of the present invention including a power supply **132** and control circuitry **134.** One or both of the power supply **132** and the control circuitry **134** may be flexible.

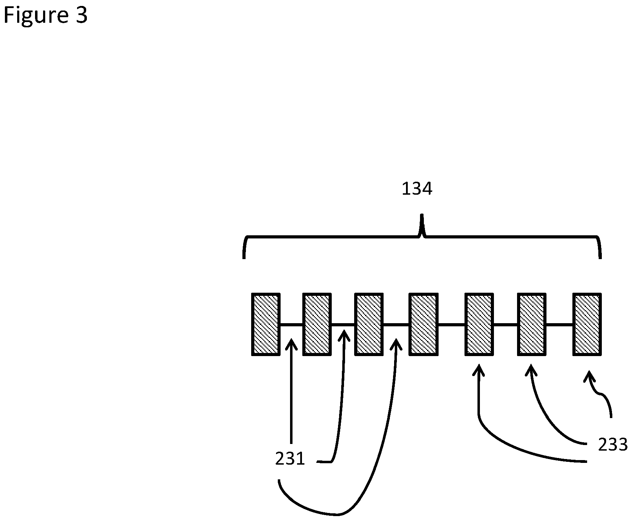

Figure 3 shows a schematic representation of the electrical components **134** of an aerosol-generating device in accordance with one configuration of the present invention including electronic modules **233** and flexible connectors **231.** At least some of the electronic modules **233** may be flexible. At least some of the electronic modules **233** may be rigid. Preferably, the electronic modules **233** are batteries.

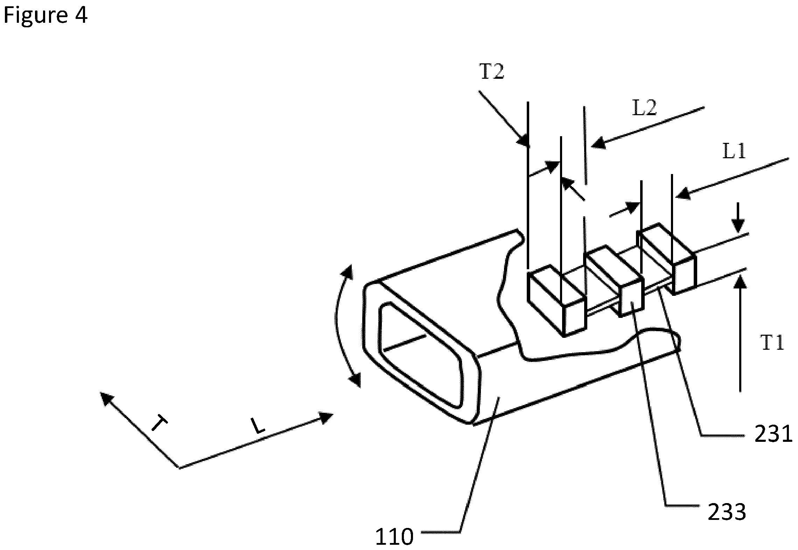

Figure 4 is a perspective cut-away view showing some components of an aerosol-generating device according to a configuration of the invention. The depicted components include a housing **110,** electronic modules **233,** and flexible connectors **231.** The flexible connectors **231** may be a ribbon wire. **"L"** in Figure 4 refers to a longitudinal direction of the device, and **"T"** refers to a transverse direction. **"L1"** in Figure 4 refers to a length of a flexible connector; **"L2"** refers to the length of an electronic module. **"T1"** in Figure 4 refers to the height or thickness of an electronic module; **"T2"** refers to the width of an electronic module

Figure 5 shows the housing of an aerosol-generating device in accordance with one configuration of the present invention. The housing has a flexible portion **(1),** a middle portion **(2),** and a mouthpiece portion **(3-4). "LA"** in Figure 5 refers to the longitudinal axis of the device when the flexible portion of the housing is in a relaxed configuration. As shown in Figure 5, when the flexible portion of the housing is in a flexed configuration, at least a portion of the flexible portion of the housing deflects from LA by an angle, **α**₁ or **α**₂. **α**₁ may be 10 degrees or more. **α**₂ may be 10 degrees or more. When the flexible portion of the housing is in the flexed configuration at least a portion of the flexible portion of the housing may deflect from LA by an angle of about 10 degrees to about 70 degrees. For example, either **α**₁ or **α**₂ or both may be about 10 degrees to about 70 degrees. The flexible portion of the housing may deflect from LA by the same amount in two or more directions or by different amounts in two or more directions.

As shown in Figure 5, the flexible portion of the housing **(1)** may have a first end, a second end, and a middle; the middle of the flexible portion of the housing is the same distance from the first end of the flexible portion of the housing as it is from the second end of the flexible portion of the housing. When the housing is in the flexed configuration, the flexible portion **(1)** may define a radius of curvature **R₁** or **R₂** at the middle of the flexed portion. **R₁** may be about 60 mm to about 100 mm or **R₂** may be about 60 mm to about 100 mm, or both **R₁** and **R₂** may be about 60 mm to about 100 mm.

The middle portion **(2)** may be rigid, at least at its core, and may include components that require solid assembly or precise fitting and interface. The middle portion **(2)** may be overmolded or coated to enable the same appearance and physical characteristics of the flexible portion **(1).**

The mouthpiece portion **(3-4)** may include a core **(4)** or a cap **(3),** or both. The cap **(3)** may be flexible, partially flexible, or rigid. The cap **(3)** may include a metallic tubular housing core **(4)** or a flexible core **(4).** The core **(4)** or the cap **(3)** may be overmolded or coated, to match the flexible portion **(1),** the middle portion **(2),** or both. The cap **(3)** may form part of the housing. The mouthpiece portion **(3-4)** may be part of a cartridge (not shown).

## Claims

1. An aerosol-generating device (100) comprising
a housing (110) having a length and defining a rigid portion along at least a portion of the length of the housing (110) and a flexible portion (120, 120a, 120b) along at least a portion of the length of the housing (110), the flexible portion (120, 120a, 120b) of the housing (110) being elastically deformable or elastically deflectable and having a relaxed or unloaded configuration and being configured to adopt a flexed or deflected configuration; and
a flexible device component disposed in the housing (110),
wherein when the housing (110) is in a relaxed or unloaded configuration, the flexible portion (120, 120a, 120b) of the housing (110) is substantially linear and the flexible device component is at least partially longitudinally aligned with the flexible portion (120, 120a, 120b) of the housing (110).

2. An aerosol-generating device (100) according to claim 1, wherein the flexible portion (120, 120a, 120b) of the housing (110) defines a longitudinal axis in the relaxed or unloaded configuration;
when the flexible portion (120, 120a, 120b) of the housing (110) is in the flexed or deflected configuration, at least a portion of the flexible portion (120, 120a, 120b) of the housing (110) deflects from the longitudinal axis by an angle of about 10 degrees or more, about 20 degrees or more, or about 30 degrees or more; and
when the flexible portion (120, 120a, 120b) of the housing (110) is in the flexed configuration, at least a portion of the flexible device component remains substantially longitudinally aligned with at least a portion of the flexible portion (120, 120a, 120b) of the housing (110) being deflected.

3. The aerosol-generating device (100) of claim 2, wherein when the flexible portion (120, 120a, 120b) of the housing (110) is in the flexed or deflected configuration, at least a portion of the flexible portion (120, 120a, 120b) of the housing (110) deflects from the longitudinal axis by an angle of about 10 degrees to about 100 degrees, or about 20 degrees to about 90 degrees, or about 30 degrees to about 80 degrees.

4. An aerosol-generating device (100) according to any one of the preceding claims, wherein the flexible portion (120, 120a, 120b) of the housing (110) has a length, and wherein the length of the flexible portion (120, 120a, 120b) of the housing (110) is about 25% to about 90% of the length of the housing (110).

5. An aerosol-generating device (100) according to any one of the preceding claims, wherein the flexible portion (120, 120a, 120b) of the housing (110) has a first end, an opposing second end, and a middle between the first end and the second end, the middle is about equal distance from the first end as it is from the second end, and in the flexed configuration the flexible portion (120, 120a, 120b) of the housing (110) defines a radius of curvature at the middle of the flexed portion, the radius of curvature being about 40 mm to about 120 mm, about 50 mm to about 100 mm, or about 60 mm to about 90 mm.

6. An aerosol-generating device (100) according to any one of the preceding claims, wherein the flexible device component comprises a flexible power supply.

7. An aerosol-generating device (100) according to claim 6, wherein the flexible power supply comprises a flexible battery.

8. An aerosol-generating device (100) according to any one of the preceding claims, wherein the flexible device component comprises flexible control circuitry.

9. An aerosol-generating device (100) according to any one of the preceding claims, wherein the flexible device component comprises at least one of a flexible storage component and a flexible fluid flow passage component.

10. The aerosol-generating device (100) of any preceding claim, wherein the rigid portion includes a heating element (140).

11. An aerosol-generating device (100) according to any one of the preceding claims, wherein the flexible device component comprises a flexible mouthpiece.

12. The aerosol-generating device (100) of any one of claims 1 to 10, wherein the rigid portion is at a first end of the aerosol-generating device (100) and includes a rigid mouthpiece and the flexible portion (120, 120a, 120b) is at a second, opposite end of the aerosol-generating device (100).

13. An aerosol-generating device (100) according to any preceding claim, wherein the aerosol-generating device (100) is a smoking article.

14. An aerosol-generating device (100) according to any preceding claim, wherein the device further comprises an aerosol-forming substrate.

15. The aerosol-generating device (100) of claim 14, wherein the aerosol-forming substrate comprises nicotine.

## Patentansprüche

1. Aerosolerzeugungsvorrichtung (100), umfassend
ein Gehäuse (110), das eine Länge aufweist und einen starren Abschnitt entlang wenigstens eines Abschnitts der Länge des Gehäuses (110) und einen flexiblen Abschnitt (120, 120a, 120b) entlang wenigstens eines Abschnitts der Länge des Gehäuses (110) definiert, wobei der flexible Abschnitt (120, 120a, 120b) des Gehäuses (110) elastisch verformbar oder elastisch auslenkbar ist und eine entspannte oder unbelastete Konfiguration aufweist und dazu ausgelegt ist, eine gebogene oder ausgelenkte Konfiguration anzunehmen; und eine in dem Gehäuse (110) angeordnete flexible Vorrichtungskomponente,
wobei, wenn das Gehäuse (110) in einer entspannten oder unbelasteten Konfiguration ist, der flexible Abschnitt (120, 120a, 120b) des Gehäuses (110) im Wesentlichen linear ist und die flexible Vorrichtungskomponente wenigstens teilweise in Längsausrichtung mit dem flexiblen Abschnitt (120, 120a, 120b) des Gehäuses (110) ausgerichtet ist.

2. Aerosolerzeugungsvorrichtung (100) nach Anspruch 1, wobei der flexible Abschnitt (120, 120a, 120b) des Gehäuses (110) eine Längsachse in der entspannten oder unbelasteten Konfiguration definiert;
wenn der flexible Abschnitt (120, 120a, 120b) des Gehäuses (110) in der gebogenen oder ausgelenkten Konfiguration ist, wenigstens ein Abschnitt des flexiblen Abschnitts (120, 120a, 120b) des Gehäuses (110) von der Längsachse um einen Winkel von etwa 10 Grad oder mehr, etwa 20 Grad oder mehr, oder etwa 30 Grad oder mehr ausgelenkt wird; und
wenn der flexible Abschnitt (120, 120a, 120b) des Gehäuses (110) in der gebogenen Konfiguration ist, wenigstens ein Abschnitt der flexiblen Vorrichtungskomponente im Wesentlichen in Längsausrichtung ausgerichtet bleibt, wobei wenigstens ein Abschnitt des flexiblen Abschnitts (120, 120a, 120b) des Gehäuses (110) ausgelenkt wird.

3. Aerosolerzeugungsvorrichtung (100) nach Anspruch 2, wobei, wenn der flexible Abschnitt (120, 120a, 120b) des Gehäuses (110) in der gebogenen oder ausgelenkten Konfiguration ist, wenigstens ein Abschnitt des flexiblen Abschnitts (120, 120a, 120b) des Gehäuses (110) von der Längsachse um einen Winkel von etwa 10 Grad bis etwa 100 Grad, oder etwa 20 Grad bis etwa 90 Grad, oder etwa 30 Grad bis etwa 80 Grad ausgelenkt wird.

4. Aerosolerzeugungsvorrichtung (100) nach einem beliebigen der vorhergehenden Ansprüche, wobei der flexible Abschnitt (120, 120a, 120b) des Gehäuses (110) eine Länge aufweist, und wobei die Länge des flexiblen Abschnitts (120, 120a, 120b) des Gehäuses (110) etwa 25 % bis etwa 90 % der Länge des Gehäuses (110) beträgt.

5. Aerosolerzeugungsvorrichtung (100) nach einem beliebigen der vorhergehenden Ansprüche, wobei der flexible Abschnitt (120, 120a, 120b) des Gehäuses (110) ein erstes Ende, ein gegenüberliegendes zweites Ende und eine Mitte zwischen dem ersten Ende und dem zweiten Ende aufweist, wobei die Mitte etwa gleich weit von dem ersten Ende entfernt ist wie von dem zweiten Ende, und in der gebogenen Konfiguration der flexible Abschnitt (120, 120a, 120b) des Gehäuses (110) einen Krümmungsradius in der Mitte des gebogenen Abschnitts definiert, wobei der Krümmungsradius etwa 40 mm bis etwa 120 mm, etwa 50 mm bis etwa 100 mm oder etwa 60 mm bis etwa 90 mm beträgt.

6. Aerosolerzeugungsvorrichtung (100) nach einem beliebigen der vorhergehenden Ansprüche, wobei die flexible Vorrichtungskomponente eine flexible Energieversorgung aufweist.

7. Aerosolerzeugungsvorrichtung (100) nach Anspruch 6, wobei die flexible Energieversorgung eine flexible Batterie aufweist.

8. Aerosolerzeugungsvorrichtung (100) nach einem der vorhergehenden Ansprüche, wobei die flexible Vorrichtungskomponente eine flexible Steuerschaltung aufweist.

9. Aerosolerzeugungsvorrichtung (100) nach einem der vorhergehenden Ansprüche, wobei die flexible Vorrichtungskomponente wenigstens eine von einer flexiblen Speicherkomponente und einer flexiblen Fluidströmungskanalkomponente aufweist.

10. Aerosolerzeugungsvorrichtung (100) nach einem beliebigen vorhergehenden Anspruch, wobei der starre Abschnitt ein Heizelement (140) beinhaltet.

11. Aerosolerzeugungsvorrichtung (100) nach einem beliebigen der vorhergehenden Ansprüche, wobei die flexible Vorrichtungskomponente ein flexibles Mundstück aufweist.

12. Aerosolerzeugungsvorrichtung (100) nach einem der Ansprüche 1 bis 10, wobei sich der starre Abschnitt an einem ersten Ende der Aerosolerzeugungsvorrichtung (100) befindet und ein starres Mundstück beinhaltet und sich der flexible Abschnitt (120, 120a, 120b) an einem zweiten, entgegengesetzten Ende der Aerosolerzeugungsvorrichtung (100) befindet.

13. Aerosolerzeugungsvorrichtung (100) nach einem beliebigen vorhergehenden Anspruch, wobei die Aerosolerzeugungsvorrichtung (100) ein Rauchartikel ist.

14. Aerosolerzeugungsvorrichtung (100) nach einem beliebigen vorhergehenden Anspruch, wobei die Vorrichtung ferner ein aerosolbildendes Substrat aufweist.

15. Aerosolerzeugungsvorrichtung (100) nach Anspruch 14, wobei das aerosolbildende Substrat Nikotin umfasst.

## Revendications

1. Dispositif de génération d'aérosol (100) comprenant
un logement (110) ayant une longueur et définissant une portion rigide le long d'au moins une portion de la longueur du logement (110) et une portion flexible (120, 120a, 120b) le long d'au moins une portion de la longueur du logement (110), la portion flexible (120, 120a, 120b) du logement (110) étant déformable élastiquement ou déviable élastiquement et ayant une configuration relâchée ou non chargée et étant configurée pour adopter une configuration fléchie ou déviée ; et
un composant de dispositif flexible disposé dans le logement (110),
dans lequel lorsque le logement (110) est dans une configuration relâchée ou non chargée, la portion flexible (120, 120a, 120b) du logement (110) est sensiblement linéaire et le composant de dispositif flexible est au moins partiellement aligné longitudinalement avec la portion flexible (120, 120a, 120b) du logement (110).

2. Dispositif de génération d'aérosol (100) selon la revendication 1, dans lequel la portion flexible (120, 120a, 120b) du logement (110) définit un axe longitudinal dans la configuration relâchée ou non chargée ;
lorsque la portion flexible (120, 120a, 120b) du logement (110) est dans la configuration fléchie ou déviée, au moins une portion de la portion flexible (120, 120a, 120b) du logement (110) dévie par rapport à l'axe longitudinal selon un angle d'environ 10 degrés ou plus, d'environ 20 degrés ou plus, ou d'environ 30 degrés ou plus ; et
lorsque la portion flexible (120, 120a, 120b) du logement (110) est dans la configuration fléchie, au moins une portion du composant de dispositif flexible reste alignée sensiblement longitudinalement avec au moins une portion de la portion flexible (120, 120a, 120b) du logement (110) qui est déviée.

3. Dispositif de génération d'aérosol (100) selon la revendication 2, dans lequel, lorsque la portion flexible (120, 120a, 120b) du logement (110) est dans la configuration fléchie ou déviée, au moins une portion de la portion flexible (120, 120a, 120b) du logement (110) dévie par rapport à l'axe longitudinal selon un angle d'environ 10 degrés à environ 100 degrés, ou d'environ 20 degrés à environ 90 degrés, ou d'environ 30 degrés à environ 80 degrés.

4. Dispositif de génération d'aérosol (100) selon l'une quelconque des revendications précédentes, dans lequel la portion flexible (120, 120a, 120b) du logement (110) a une longueur, et dans lequel la longueur de la portion flexible (120, 120a, 120b) du logement (110) est d'environ 25 % à environ 90 % de la longueur du logement (110).

5. Dispositif de génération d'aérosol (100) selon l'une quelconque des revendications précédentes, dans lequel la portion flexible (120, 120a, 120b) du logement (110) a une première extrémité, une deuxième extrémité opposée, et un milieu entre la première extrémité et la deuxième extrémité, le milieu est environ à la même distance de la première extrémité qu'il l'est de la deuxième extrémité, et dans la configuration fléchie la portion flexible (120, 120a, 120b) du logement (110) définit un rayon de courbure au milieu de la portion fléchie, le rayon de courbure étant d'environ 40 mm à environ 120 mm, d'environ 50 mm à environ 100 mm, ou d'environ 60 mm à environ 90 mm.

6. Dispositif de génération d'aérosol (100) selon l'une quelconque des revendications précédentes, dans lequel le composant de dispositif flexible comprend une alimentation électrique flexible.

7. Dispositif de génération d'aérosol (100) selon la revendication 6, dans lequel l'alimentation électrique flexible comprend une pile flexible.

8. Dispositif de génération d'aérosol (100) selon l'une quelconque des revendications précédentes, dans lequel le composant de dispositif flexible comprend une circuiterie de commande flexible.

9. Dispositif de génération d'aérosol (100) selon l'une quelconque des revendications précédentes, dans lequel le composant de dispositif flexible comprend au moins l'un parmi un composant de stockage flexible et un composant de passage d'écoulement de fluide flexible.

10. Dispositif de génération d'aérosol (100) selon l'une quelconque des revendications précédentes, dans lequel la portion rigide comporte un élément de chauffage (140).

11. Dispositif de génération d'aérosol (100) selon l'une quelconque des revendications précédentes, dans lequel le composant de dispositif flexible comprend un embout buccal flexible.

12. Dispositif de génération d'aérosol (100) selon l'une quelconque des revendications 1 à 10, dans lequel la portion rigide est à une première extrémité du dispositif de génération d'aérosol (100) et comporte un embout buccal rigide et la portion flexible (120, 120a, 120b) est à une deuxième extrémité opposée du dispositif de génération d'aérosol (100).

13. Dispositif de génération d'aérosol (100) selon l'une quelconque des revendications précédentes, dans lequel le dispositif de génération d'aérosol (100) est un article à fumer.

14. Dispositif de génération d'aérosol (100) selon l'une quelconque des revendications précédentes, dans lequel le dispositif comprend en outre un substrat formant aérosol.

15. Dispositif de génération d'aérosol (100) selon la revendication 14, dans lequel le substrat formant aérosol comprend de la nicotine.
